# EUROPÄISCHE PATENTANMELDUNG

(11) **EP 1 197 203 A1**
(43) Veröffentlichungstag der Anmeldung: **17.04.2002**
(21) Anmeldenummer: 01123315.2
(22) Anmeldetag: 08.10.2001
(51) Int. Cl.: A61K 7/13

(54) **Haarfärbemittel**

(30) Priorität: 14.10.2000 DE 10051003
(71) Anmelder: GOLDWELL GmbH, 64280 Darmstadt (DE)
(72) Erfinder: Lorenz, Heribert, 64401 Gross Bieberau (DE); Kufner, Frank, 64297 Darmstadt (DE)

(57) **Zusammenfassung**

Ein Haarfärbemittel auf Basis eines mit Peroxid reagierenden Oxidationsfarbstoff-Vorprodukts enthaltend 3,4-Diamino-5-hydroxypyrazol und/oder dessen wasserlösliche Salze und mindestens eine weitere Entwickler- und/oder Kupplersubstanz, ausgewählt aus der Gruppe 2,5-Diaminotoluol, 1,4-Diaminobenzol, einem Hydroxytriaminopyrimidin wie 2,5,6-Triamino-4-hydroxypyrimidin, 1-Methyl-2-hydroxy-4-aminobenzol, 3-Aminophenol, 4-Aminophenol, 2-Amino-3-hydroxypyridin, 1-Naphthol, 1-Methoxy-2-amino-4-(β-hydroxyethylamino)benzol, 2-(2'-Hydroxyethylamino)-5-aminotoluol, 1-Amino-4-bis-(2'-hydroxyethyl)-aminobenzol, 1-(β-Hydroxyethyl)-2,5-diaminobenzol, 5-Amino-2-methoxyphenol und/oder 4-Amino-3-methylphenol bzw. deren wasserlöslichen Salzen.

## Beschreibung

Die vorliegende Erfindung betrifft ein Haarfärbemittel auf Basis eines mit Peroxid reagierenden Oxidationsfarbstoff-Systems, das dauerhafte intensive Farbtöne liefert, die entweder als solche angewandt werden, oder, in Kombination mit weiteren Entwickler- und/oder Kupplersubstanzen, zur Erzielung weiterer Farbnuancen benutzt werden können und das Haar selbst bei kurzfristiger wiederholter Anwendung nicht schädigt.

Die nach wie vor in Haarfärbemitteln meist eingesetzten Entwicklersubstanzen sind 1,4-Diaminobenzol (p-Phenylendiamin) und 1-Methyl-2,5-diaminobenzol (p-Toluylendiamin). Die Verwendung dieser Substanzen wird den farbtechnischen Wünschen der Anwender zwar weitgehend gerecht, es gibt jedoch immer noch Farbnuancen, die dadurch nicht voll erreicht werden können.

Es wurde auch bereits vorgeschlagen, diese Lücke durch Verwendung alternativer Entwicklersubstanzen zu schließen. Dies ist in beschränktem Umfang möglich durch den Einsatz von Tetraaminopyrimidin oder 2-(2,5-Diaminophenyl)ethanol (vgl. EP-A 7537 und EP-B 400 330); jedoch müssen dann Abstriche in der Farbintensität anderer Nuancen hingenommen werden.

Eine weitgehend optimale Lösung dieses Problems wird durch den in der EP-A 615 743 beschriebenen Einsatz von 2-(2'-Hydroxyethylamino)-5-aminotoluol bzw. dessen wasserlöslichen Salzen und die aus der EP-B 467 026 bekannten Triaminohydroxypyrimidine, insbesondere 2,5,6-Triamino-4-hydroxypyrimidin, 2,4,5-Triamino-6-hydroxypyrimidin, 4,5,6-Triamino-2-hydroxypyrimidin bzw. deren Salze, insbesondere die Sulfate, als Entwicklersubstanzen in Haarfärbemitteln erreicht.
Selbst dadurch bleiben jedoch noch farbtechnische Wünsche offen.

Auch 3,4-Diamino-5-hydroxypyrazol ist bereits als Bestandteil von Oxidations-Haarfärbemitteln vorgeschlagen worden.
Es war jedoch bisher nicht möglich, Färbungen im Gelb-, Orange- und Beigebereich auf diese Weise herzustellen.

Die Erfindung geht von der Aufgabe aus, diesem Mangel abzuhelfen, und Oxidationshaarfärbemittel, die 3,4-Diamino-5-hydroxypyrazol bzw. dessen wasserlösliche Salze enthalten, zur Verfügung zu stellen, die intensive, glänzende Färbungen insbesondere im Gelb-, Orange- und Beigebereich, liefern, auf denen sich, durch Zusatz weiterer Entwickler- und Kupplersubstanzen, auch andere Farbnuanciereungen aufbauen lassen.

Diese Aufgabe wird dadurch gelöst, daß ein solches Haarfärbemittel ein mit Peroxid reagierendes Oxidationsfarbstoff-System enthält, das aus einer Kombination aus 3,4-Diamino-5-hydroxypyrazol bzw. dessen wasserlöslichen Salzen und mindestens einer weiteren Kuppler- und/oder Entwicklersubstanz, ausgewählt aus mindestens einer Substanz der Gruppe 2,5-Diaminotoluol, 1,4-Diaminobenzol, einem Hydroxytriaminopyrimidin wie 2,5,6-Triamino-4-hydroxypyrimidin, 1-Methyl-2-hydroxy-4-aminobenzol, 3-Aminophenol, 4-Aminophenol, 2-Amino-3-hydroxypyridin, 1-Naphthol, 1-Methoxy-2-amino-4-(β-hydroxyethylamino)benzol, 2-(2'-Hydroxyethylamino)-5-aminotoluol, 1-Amino-4-bis-(2'-hydroxyethyl)aminobenzol, 1-(β-Hydroxyethyl)-2,5-diaminobenzol, 5-Amino-2-methoxyphenol und/oder 4-Amino-3-methylphenol bzw. deren wasserlöslichen Salzen, enthält.

Zusätzlich zu den genannten Entwickler- und Kupplersubstanzen können noch weitere eingesetzt werden.
Diese sind beispielsweise ausgewählt aus der Gruppe 2,5-Diaminopyridin, 2,6-Diaminopyridin, 3-Amino-2-methylamino-6-methoxypyridin, 2-(Dimethylamino)-5-aminopyridin, 2,4,5,6-Tetraaminopyrimidin, 2-Amino-5-N,N-diethylaminotoluol, 1,3-Diaminobenzol, Resorcin, 2-Methylresorcin, 4-Chlorresorcin, 2-Amino-4-Chlorphenol, 1,6-Dihydroxynaphthalin, 1,7-Dihydroxynaphthalin, 2-Aminophenol, 2-Methyl-5-hydroxyethylaminophenol, 5-Amino-2-methylphenol und/oder, 2-Amino-4-β-hydroxyethylaminoanisol bzw. deren wasserlösliche Salzen.
Zusätzlich können in den erfindungsgemäßen Zusammensetzungen weitere Entwickler- und Kupperlersubstanzen enthalten sein.

Bei Anwendung dieser Zusammensetzungen auf Basis einer üblichen Grundlage werden nach der Oxidation mit Peroxid sehr ausdrucksvolle, intensive, dauerhafte Haarfärbungen vor allem im Gelb-, Beige- und Goldbereich erhalten, die durch Zusatz entsprechender weiterer Entwickler- und Kupplersubstanzen noch zu anderen Farbnuancen variiert werden können.

Das 3,4-Diamino-5-hydroxypyrazol liegt vorzugsweise als wasserlösliches Salz, insbesondere als Sulfat, vor.

Auch die zusätzliche Mitverwendung weiterer, an sich bekannter Entwicklersubstanzen ist möglich. Neben den bereits oben genannten sind hierbei insbesondere noch 5-Aminosalicylsäure und/oder 1,2,4-Triaminobenzol zu erwähnen.

Die Gesamtkonzentration der Entwicklersubstanzen liegt üblicherweise zwischen etwa 0,05 und 5 %, vorzugsweise 0,1 und 4 %, insbesondere 0,25 bis 0,5 % und 2,5 bis 3 % Gew.-% der Gesamtzusammensetzung des Haarfärbemittels (ohne Oxidationsmittel), wobei sich die Angaben jeweils auf den Anteil an freier Base beziehen.

Das bevorzugte Gewichtsverhältnis von 3,4-Diamino-5-hydroxypyrazol zu den weiteren Entwickler- und Kupplersubstanzen liegt dabei zwischen etwa 1 : 8 bis 8 : 1, vorzugsweise etwa 1 : 5 bis 5 : 1, insbesondere 1 : 2 bis 2 : 1.

Die Kupplersubstanz(en) als Reaktionspartner der Entwicklersubstanz(en) liegen in den erfindungsgemäßen Haarfärbemitteln etwa im gleichen molaren Anteil wie die Entwicklersubstanzen vor, d. h., also in Mengen von 0,05 bis 5,0 %, vorzugsweise 0,1 bis 4 %, insbesondere 0,5 bis 3 Gew.-% der Gesamtzusammensetzung (ohne Oxidationsmittel), wobei sich die Angaben jeweils auf den Anteil an freier Base beziehen.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.

Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemittels, d. h. nach Vermischung mit Peroxid, kann sowohl im schwach sauren, d. h. einem Bereich von 5,5 bis 6,9, im neutralen als auch im alkalischen Bereich, d. h. zwischen pH 7,1 und 10 liegen.

Im folgenden werden verschiedene Ausführungsbeispiele zur Erläuterung der Erfindung gegeben.

| Grundlage | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | - 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfum | 0,4 |
| Ammoniak, 25%ig | 1,0 |
| Ammoniumchlorid | 0,5 |
| Panthenol | 0,8 |
| Wasser | @ 100,00 |

Die erfindungsgemäßen Oxidationsfarbstoff-Kombinationen wurden, unter entsprechender Verringerung des Wassergehalts, in diese Grundlage eingearbeitet.

Die Ausfärbungen erfolgten jeweils an Woll-Läppchen und Strähnen aus gebleichtem Menschenhaar, durch Aufbringen einer 1:1-Mischung aus Farbstoff-Vorprodukt und 6%iger Wasserstoffperoxid-Lösung (pH-Wert der Mischung: 9,8) und zwanzigminütiger Einwirkung bei Zimmertemperatur, folgendem Auswaschen und Trocknen.

Es wurden die folgenden Färbungen erzielt:

| Beispiel 1: | | |
|---|---|---|
| 0,48 | (Gew.-%) | 3,4-Diamino-5-hydroxypyrazolsulfat (PC 58) |
| 0,28 | | 1-Methyl-2-hydroxy-4-aminobenzol |

| Färbung: | | |
|---|---|---|
| | Intensives Hellgold. | |

| Beispiel 2: | | |
|---|---|---|
| 0,48 | (Gew.-%) | PC 58 |
| 0,31 | | 5-Amino-2-methoxyphenol |

| Färbung: | | |
|---|---|---|
| | Kräftiges Orangebeige. | |

| Beispiel 3: | | |
|---|---|---|
| 0,48 | (Gew.-%) | PC 58 |
| 0,50 | | p-Toluylendiaminsulfat |

| Färbung: | | |
|---|---|---|
| | Intensives Mittelbraunbeige. | |

| Beispiel 4: | | |
|---|---|---|
| 0,48 | (Gew.-%) | PC 58 |
| 0,63 | | 1-Methoxy-2-amino-4-(β-hydroxyethylamino)-benzolsulfat |

| Färbung: | | |
|---|---|---|
| | Intensives Hellbraunbeige. | |

| Beispiel 5: | | |
|---|---|---|
| 0,48 | (Gew.-%) | PC 58 |
| 0,32 | | 1-Naphthol |

| Färbung: | | |
|---|---|---|
| | Glänzendes Hellgoldgelb. | |

| Beispiel 6: | | |
|---|---|---|
| 0,48 | (Gew.-%) | PC 58 |
| 0,25 | | 4-Aminophenol |

| Färbung: | | |
|---|---|---|
| | Intensives Hellgoldbraun. | |

| Beispiel 7: | | |
|---|---|---|
| 0,48 | (Gew.-%) | PC 58 |
| 0,54 | | 4-Hydroxy-2,5,6-triaminopyridinsulfat |

| Färbung: | | |
|---|---|---|
| | Glänzendes Hellgelb. | |

## Patentansprüche

1. Haarfärbemittel auf Basis eines mit Peroxid reagierenden Oxidationsfarbstoff-Vorprodukts, enthaltend 3,4-Diamino-5-hydroxypyrazol und/oder dessen wasserlösliche Salze und mindestens eine weitere Entwickler- und/oder Kupplersubstanz, ausgewählt aus der Gruppe 2,5-Diaminotoluol, 1,4-Diaminobenzol, einem Hydroxytriaminopyrimidin wie 2,5,6-Triamino-4-hydroxypyrimidin, 1-Methyl-2-hydroxy-4-aminobenzol, 3-Aminophenol, 4-Aminophenol, 2-Amino-3-hydroxypyridin, 1-Naphthol, 1-Methoxy-2-amino-4-(β-hydroxyethylamino)benzol, 2-(2'-Hydroxyethylamino)-5-aminotoluol, 1-Amino-4-bis-(2'-hydroxyethyl)-aminobenzol,1-(β-Hydroxyethyl)-2,5-diaminobenzol, 5-Amino-2-methoxyphenol und/oder 4-Amino-3-methylphenol bzw. deren wasserlöslichen Salze enthält.

2. Haarfärbemittel nach Anspruch 1, enthaltend als weitere Entwickler- und Kupplersubstanz(en) mindestens eine Komponente, ausgewählt aus der Gruppe 2,5-Diaminopyridin, 2,6- Diaminopyridin, 3-Amino-2-methylamino-6-methoxypyridin, 2-(Dimethylamino)-5-aminopyridin, 2,4,5,6-Tetraaminopyrimidin, 5-Amino-2-methylphenol, 2-Methyl-5-hydroxyethylaminophenol, 2-Amino-5-N,N-diethylaminotoluol, 2-Amino-4-Chlorphenol, 1,4-Diaminobenzol, 1,3-Diaminobenzol, 1,6-Dihydroxynaphthalin, 1,7 Dihydroxynaphthalin, Resorcin, 2-Methylresorcin, 4-Chlorresorcin, 2-Aminophenol, 5-Amino-2-methoxyphenol, und/oder 2-Amino-4-β-hydroxyethylaminoanisol bzw. deren wasserlöslichen Salzen.
